# EUROPEAN PATENT APPLICATION

(11) **EP 1 563 832 A1**
(43) Date of publication of application: **17.08.2005**
(21) Application number: 05010025.4
(22) Date of filing: 20.04.2000
(51) Int. Cl.: A61K 9/127, A61K 47/48, A61K 39/395, A61P 35/00

(54) **Liposome comprising a compound containing 2 polyalkylene glycol groups**

(30) Priority: 23.04.1999 JP 11573799; 23.04.1999 JP 11573899
(62) Divisional of application: 00917390.7
(71) Applicant: Mitsubishi Chemical Corporation, Tokyo 108-0014 (JP)
(72) Inventor: Tagawa, Toshiaki c/o Mitsubishi-Tokyo Pharmac.,Inc, Yokohama-shi, kanagawa 227-0033 (JP); Hosokawa, Saiko c/o Mitsubishi-Tokyo Pharmac.,Inc, Yokohama-shi, kanagawa 227-0033 (JP)
(74) Representative: TER MEER STEINMEISTER & PARTNER GbR

(57) **Abstract**

A liposome wherein a compound containing two polyalkylene glycol groups is bonded through a thioether group to a liposome comprising lipids whose partial component has maleimidated terminal, and wherein an amount of the bonded compound is 15 to 30 mole% based on one mole of the maleimidated lipid contained in the liposome. The liposome achieves both excellent blood retention and therapeutic effect.

## Description

### Technical Field

The present invention relates to a liposome. More specifically, the present invention relates to a liposome bonded with an antibody and/or polyalkylene glycol, which has superior blood retention and therapeutic effect.

### Background Art

As means for transporting a large amount of a drug to a specific site, a method has been proposed which includes the step of encapsulating a drug in a liposome and bonding an antibody to surface of the liposome. In the field of cancer treatment, in particular, a number of reports have been made on efficacy of an antibody-bonded liposome in which an antitumor agent is encapsulated (Konno et al., Cancer Tes., 47, 4471, 1987; Japanese Patent Unexamined Publication (Kokai) No. 58-134032). Moreover, a method of bonding polyethylene glycol to a liposome has been proposed as a method for solving problems with a liposome, i.e., leakage of encapsulated substances, agglutination of liposomes, capture in reticuloendotherial organs and the like (Japanese Patent Unexamined Publication (Kokai) Nos. 1-249717 and 2-149512; Klibanovet, A.L. et al., FEBS Lett., 268, 235, 1990).

Japanese Patent Unexamined Publication (Kokai) No. 4-346918 discloses a protein-bonded liposome encapsulating a drug, wherein the liposome is characterized to have a protein and a residue of compound containing a polyalkylene glycol moiety both of which are bound through their respective thiol groups to maleimide groups on the surface of the liposome encapsulating a drug. This liposome is produced by reacting a thiol group bonded to the antibody and a thiol group bonded to the compound containing a polyalkylene glycol moiety with the maleimide groups on the liposome surface. The liposome has a feature in that non-specific uptake in the reticuloendotherial system, such as in liver and spleen as observed in conventional liposomes, is suppressed and selective chemotherapy can be achieved.

In the above patent publication, any specific amount of the compound containing a polyalkylene glycol moiety bound to the liposome is not explained. The document, however, discloses that 0.1 to 20 mole % of thiolated antibodies are reacted with 1 mole of maleimide groups (maleimidated lipids), and then an excessive amount of, preferably two or more fold amount of the compound containing a thiolated polyalkylene glycol moiety based on the remaining maleimide groups is added to obtain an antibody-bonded liposome modified with polyalkylene glycol (Paragraph 0016). In the method for preparing a liposome specifically described in the examples, 5 micromole of a compound containing a thiolated polyethylene glycol moiety is reacted with 100 mg of total lipids (corresponding to 3.1 mole% to the total lipids). It is considered that a liposome modified with a theoretical amount of polyethylene glycol (i.e., the amount of the maleimide groups remaining on the liposome surface) was obtained by reacting a large excessive amount of the compound containing a thiolated polyethylene glycol moiety based on the lipids.

Further, in the above patent publication, any specific amount of the antibodies bound to the liposome is not explained. The publication explains that 0.1 to 20 mole% of the thiolated antibodies were reacted with 1 mole of maleimide group of the maleimidated lipid (corresponding to 0.3 to 60 mg per 100 mg of the total lipids). In the method for preparing the liposome specifically described in the examples, 5 mg of the antibodies were bonded to 100 mg of the total lipids (corresponding to 1.7 mole% based on the maleimidated lipids). However, the above publication contains no specific disclosure as to an amount of bonding other than the above.

### Disclosure of the Invention

On the basis of the liposome described in Japanese Patent Unexamined Publication (Kokai) No. 4-346918, the inventors of the present invention conducted various studies to provide a liposome having higher blood retention and superior safety. As a result, it was surprisingly found that, when an amount of polyalkylene glycol that modifies the liposome was reduced below the theoretical value (i.e., the amount of the maleimide groups remaining on the liposome surface) in the liposome described in Japanese Patent Laid-Open Publication (Kokai) No. 4-346918, almost the same level of blood retention as that of the known liposome was obtained by the liposome having a modification amount less than the theoretical value.

Moreover, on the basis of the liposome described in Japanese Patent Unexamined Publication (Kokai) No. 4-346918, the inventors of the present invention further conducted studies to provide a liposome that can achieve a higher therapeutic effect. As a result, it was surprisingly found that, when the amount of the bonded antibodies of the liposome described in Japanese Patent Unexamined Publication (Kokai) No. 4-346918 was reduced, much higher therapeutic effect was achieved than that of the liposome described in the examples of the above publication. The present invention was achieved on the basis of these findings.

The present invention thus provides a liposome wherein a compound containing a polyalkylene glycol moiety is bonded through a thioether group to a liposome comprising lipids whose partial component has maleimidated terminal, wherein an amount of the bonded compound is 15 to 50 mole% based on one mole of the maleimidated lipid contained in the liposome; the aforementioned liposome wherein the amount of the bonded compound is 15 to 30 mole% based on one mole of the maleimidated lipids; the aforementioned liposome which is obtained by reacting a maleimide group in the maleimidated lipid with the compound containing a polyalkylene glycol moiety introduced with a thiol group; the aforementioned liposome wherein the compound is bonded to a surface of the liposome; the aforementioned liposome wherein the polyalkylene glycol is polyethylene glycol; the aforementioned liposome wherein the compound has two polyethylene glycol groups; the aforementioned liposome wherein the polyethylene glycol has a molecular weight of 2,000 to 7,000 Da; the aforementioned liposome wherein the polyethylene glycol has a molecular weight of about 5,000 Da; and the aforementioned liposome wherein an antibody is further boned on a surface of the liposome.

The present invention further provides a liposome wherein an antibody is bonded through a thioether group to a liposome comprising lipids whose partial component has maleimidated terminal, wherein an amount of the bonded antibody is 0.1 to 2 mole% based on one mole of the maleimidated lipids contained in the liposome; the aforementioned liposome wherein an amount of the bonded antibody is 0.4 to 0.7 mole% based on one mole of the maleimidated lipids; the aforementioned liposome which is obtained by reacting a liposome having a maleimide group and a sulfur-containing group deriving from the antibody to form a thioether bond; the aforementioned liposome wherein the antibody is a GAH antibody; the aforementioned liposome wherein the antibody is a F(ab')₂ antibody fragment; and the aforementioned liposome wherein a compound containing a polyalkylene glycol moiety is further bonded to a surface of the liposome.

The present invention also provides a liposome wherein a compound containing a polyalkylene glycol moiety and an antibody are bonded through thioether groups to a liposome comprising lipids whose partial component has maleimidated terminal, wherein an amount of the bonded compound is 15 to 30 mole% and an amount of the bonded antibody is 0.4 to 0.7 mole% based on one mole of the maleimidated lipids contained in the liposome; the aforementioned liposome which is obtained by reacting a maleimide group in the maleimidated lipid with the compound containing a polyalkylene glycol moiety introduced with a thiol group; the aforementioned liposome wherein the compound is bonded to a surface of the liposome; the aforementioned liposome wherein the polyalkylene glycol is polyethylene glycol; the aforementioned liposome wherein the compound has two polyethylene glycol groups; the aforementioned liposome wherein the polyethylene glycol has a molecular weight of 2,000 to 7,000 Da; the aforementioned liposome wherein the polyethylene glycol has a molecular weight of about 5,000 Da; the aforementioned liposome which is a liposome obtained by reacting a liposome having a maleimide group and a sulfur-containing group deriving from the antibody to form a thioether bond; the aforementioned liposome wherein the antibody is a GAH antibody; and the aforementioned liposome wherein the antibody is a F(ab')₂ antibody fragment.

The present invention also provides the aforementioned liposome, which is an anticancer agent; the aforementioned anticancer agent wherein the cancer is stomach cancer or colon cancer; and a method for treating cancer by using the aforementioned liposome.

### Brief Description of the Drawings

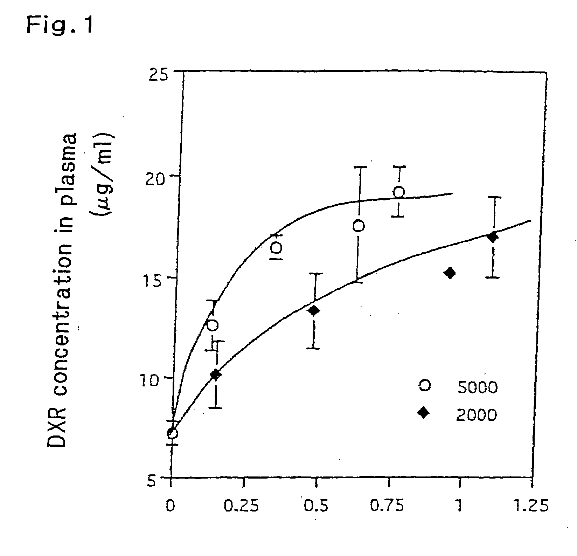

Fig. 1 shows correlation between the amount of polyethylene glycol bonded to a liposome (encapsulating doxorubicin (DXR) and bonded with no antibody) and the DXR concentration in plasma. The horizontal axis indicates the PEG amount (in mole% based on one mole of DPPC). The PEG amount in mole% based on the total lipid and maleimidated DPPE are 0.16 and 8.9 (0.25); 0.32 and 17.8 (0.5); 0.48 and 26.7 (0.75); 0.64 and 35.6 (1) and 0.81 and 45 (1.25), respectively (the parenthesized values indicate mole% based on one mole of DPPC).

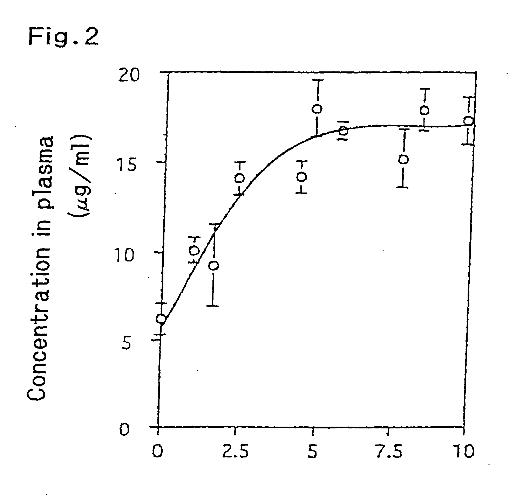

Fig. 2 shows correlation between the amount of polyethylene glycol bonded to a liposome (encapsulating DXR and bonded with antibodies) and the DXR concentration in plasma. The horizontal axis indicates the PEG amount (the PEG amount (mg) per 100 mg of lipids). The PEG amounts in mole% based on one mole of DPPC, total lipids and maleimidated DPPE are 0.25, 0.16 and 8 (2.5); 0.50, 0.31 and 17 (5); 0.75, 0.47 and 26 (7.5); and 1.0, 0.62 and 34 (10), respectively (the parenthesized values indicate the PEG amounts (mg) per 100 mg of lipids).

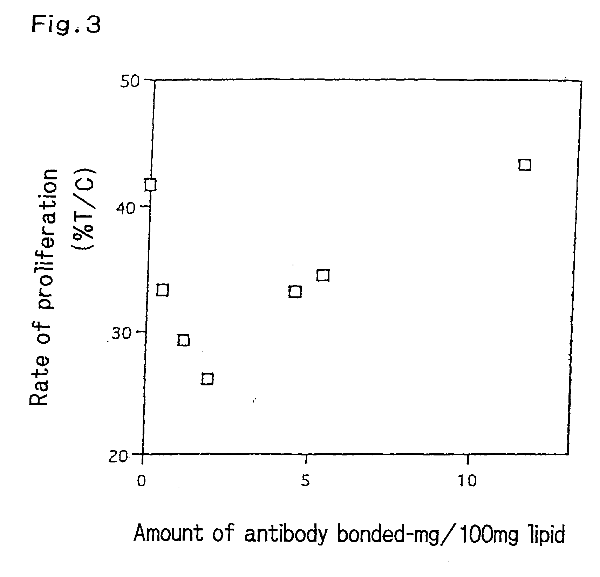

Fig. 3 shows correlation between the amount of antibodies bonded to a liposome (encapsulating DXR) and tumor inhibitory effect (% T/C).

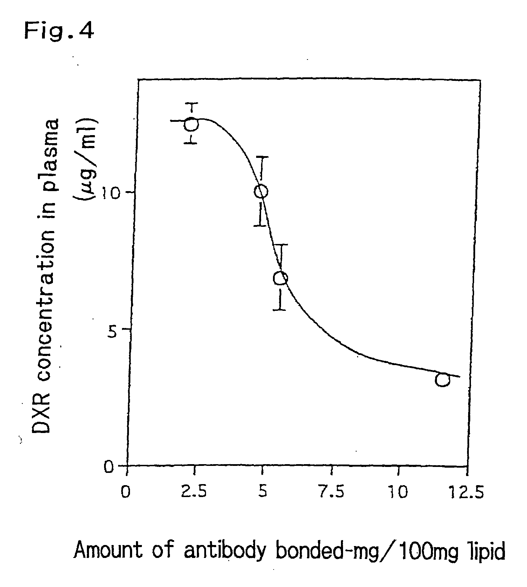

Fig. 4 shows correlation between the DXR amount in plasma (blood retention) and the amount of bonded antibodies in samples in which the amount of the bonded antibodies is 2 mg or more per 100 mg of lipids.

### Best Mode for Carrying out the Invention

Examples of lipids constituting the liposome of the present invention include natural lecithins (for example, egg yolk lecithin and soybean lecithin), phospholipids such as dipalmitoylphosphatidylcholine (DPPC), dimyristoylphosphatidylcholine (DMPC), distearoylphosphatidylcholine (DSPC), dioleoylphosphatidylcholine (DOPC), dimyristoylphosphatidylethanolamine (DMPE), dipalmitoylphosphatidylethanolamine (DPPE), dioleoylphosphatidylethanolamine (DOPE), dipalmitoylphosphatidic acid (DPPA), dipalmitoylphosphatidyl glycerol (DPPG) and dimyristoylphosphatidic acid (DMPA), glycolipids such as sphingoglycolipids and glyceroglycolipids, fatty acids, dialkyldimethylammnonium amphiphiles, polyglycerol alkyl ethers and polyoxyethylene alkyl ethers (Liposome Technology, 2nd edition, vol. 1, 141, 1993), alkylglycosides, alkylmethylglucamide, alkyl sucrose esters, dialkylpolyoxyethylene ether, dialkylpolyglycerol ether and so forth (Liposome Technology, 2nd edition, vol. 1, 141, 1993), amphipathic block copolymers such as polyoxyethylene-polylactic acid (International Patent Unexamined Publication in Japanese (Kohyo) No. 6-508831) and the like. However, lipids are not limited to these examples. These lipids may be used alone or in combination of two or more kinds, and can also be used in combination with a non-polar substance such as cholesterol or a cholesterol derivative such as DC-chol (3β-[N-(N',N'-dimethylaminoethyl)carbamoyl]cholesterol).

In the liposome of the present invention, a maleimidated lipid (hereinafter referred to as "maleimidated lipid") such as maleimidated phosphatidylethanolamine is required to be used as a part of the lipid components for bonding of a compound containing polyalkylene glycol and, as required, a protein such as antibodies. A ratio of the maleimidated lipid to the total lipids is usually about 0.5 to 10 mole%.

Maleimidated phosphatidylethanolamine will be explained as an example. This compound is obtained by reacting a maleimido-containing compound having reactivity with an amino group of phosphatidylethanolamine (PE). The maleimido-containing compound may contain a residue such as a caproyl group, a benzoyl group or a phenylbutyryl group. Examples of such a compound include N-(e-maleimidocaproyloxy)succinimide, N-succinimidyl-4-(p-maleimidophenyl)-butylate, N-succinimidyl-4-(p-maleimidophenyl)propionate, N-(γ-maleimido-butyryloxy)succinimide and the like. As PE, phosphatidylethanolamines such as dipalmitoylphosphatidylethanolamine (DPPE), dimyristoylphosphatidylethanolamine (DMPE) or dioleoylphosphatidylethanolamine (DOPE) can be used, and DPPE is preferred. Further, a charged substance such as stearylamine or dicetylphosphate may also be contained as a lipid component. The liposome of the present invention may be a fusiogenic liposome incorporating a part or whole of virus, for example, a liposome fused with Sendai virus.

For a typical liposome, for example, a lipid composition containing 0.3 to 1 mole, preferably, 0.4 to 0.6 mole of cholesterol and 0.01 to 0.2 mole, preferably 0.02 to 0.1 mole, more preferably 0.02 to 0.05 mole, of maleimidated phosphatidylethanolamine per mole of phosphatidylcholine can be used. When phosphatidic acid is added, a lipid composition containing 0.4 mole or less, preferably 0.15 mole or less of phosphatidic acid can be used.

Methods for preparing the liposome of the present invention are not particularly limited and any method available to those skilled in the art can be used. Further, a structure of the liposome of the present invention is not particularly limited and the liposome may be in any structure. For example, the liposome may be any of a multilamellar liposome (MLV) obtained by adding an aqueous solution to a thin lipid membrane formed on a glass wall and subjecting the membrane to mechanical shaking; a small unilamellar liposome (SUV) obtained by the sonication method, the ethanol injection method or the French press method; and a large unilamellar liposome (LUV) obtained by the surfactant removing method, the reversed-phase evaporation method (Liposome, Sunamoto J. et al., Nankodo Co., Ltd., 1998), or the extrusion method in which MLV is extruded through a membrane having a uniform pore size by pressurization (Liposome Technology, 2nd edition, vol.1, 141, 1993). The particle diameter of the liposome is, for example, 300 nm or smaller, preferably, about 30 to 200 nm.

A medicament can be encapsulated in the liposome of the present invention. Types of the medicament are not particularly limited. For example, usable medicaments include antitumor agents such as doxorubicin (adriamycin), daunomycin, vinblastine, cisplatin and 5-fluorouracil (5-FU); adrenalin blocking agents such as timolol; antihypertensive agents such as clonidine; antiemetic drugs such as procainamide; antimalarial agents such as chloroquinine; and pharmaceutically acceptable salts and derivatives thereof; radioactive substances such as rhenium 186, iodine 131 and yttrium 90; physiologically active substances such as ricin A, diphtheria toxin and TNF and DNA encoding the substance and the like. However, medicaments that can be encapsulated in the liposome of the present invention are not limited to these examples.

As pharmaceutically acceptable salts of the aforementioned medicaments, preferred examples include salts with pharmaceutically acceptable multivalent anionic substances such as citrates, tartrates, glutamates, and salts with derivatives thereof. As the medicament encapsulated in the liposome of the present invention, antitumor agents are preferred. In addition to a therapeutic medicament, a medicament for diagnosis can be encapsulated in the liposome of the present invention. Examples of the diagnostic medicament include radioactive isotopes, for example, imaging agents such as indium and technetium; contrast media such as iodine and gadolinium; enzymes such as horseradish peroxidase, alkaline phosphatase and β-galactosidase; fluorescent substances such as europium derivatives; luminescent substances such as N-methylacridium derivatives and the like. However, medicaments are not limited to these examples.

Methods for encapsulating the medicaments into the liposome are not particularly limited, and any method available to those skilled in the art can be applied. For example, upon formulation of a liposome, a medicament can be added as an aqueous solution and encapsulated in the liposome. Further, after formulation of the liposome, a method can be applied in which a concentration gradient such as a pH gradient is formed across a vesicle and this potential is used as a driving force to incorporate an ionizable drug into the liposome (Cancer Res., 49, 5922, 1989; BBA, 455, 269, 1976).

The liposome of the present invention is characterized in that the liposome is modified with a compound containing a polyalkylene glycol moiety and further modified with a specific amount of an antibody. As the polyalkylene glycol, for example, polyethylene glycol (PEG), polypropylene glycol or the like can be used, and polyethylene glycol is preferred. When polyethylene glycol is used, those having a molecular weight of about 2,000 to 7,000 Da, preferably about 5,000 Da, can be used.

The liposome of the present invention is in a structure wherein the compound containing a polyalkylene glycol is bonded through a thioether bond to a maleimidated lipid on the liposome surface. The liposome bonded with polyalkylene glycol can usually be prepared by introducing a thiol group into a compound containing polyalkylene glycol and then reacting the compound with a maleimide group on a liposome. Examples of the compound containing polyalkylene glycol generally include a compound that has a polyethylene glycol group and a terminal that can be thiolated or a compound having a mercapto group at a terminal thereof. Specifically, examples include a compound in which a polyalkylene glycol group is bonded to triazine and a compound in which the triazine is further substituted with an amino acid or the like. The compound may have two polyalkylene glycol groups (two-chain type).

When polyethylene glycol is used as the polyalkylene glycol, applicable methods include, for example, a method of condensing monomethoxypolyoxyethyleneamine and any of various thiolcarboxylic acids by dehydration; a method of introducing a pyridyldithiopropionyl group into monomethoxypolyoxyethyleneamine using SPDP and further reducing the product; a method of introducing a thiol group into monomethoxypolyoxyethyleneamine by using iminothiolane; a method of bonding an active ester of monomethoxypolyoxyethylenecarboxylic acid and any of various thiolamines; a method of condensing a polyethylene glycol triazine derivative with a thiolamine or the like. More specifically, a cysteine-bonded activated PEG 2 can be obtained by reacting 2,4-bis(polyethylene glycol)-6-chloro-s-triazine (activated PEG 2, Seikagaku Corporation) with cystine and further reducing the product.

An introduced amount of the compound containing a polyalkylene glycol moiety in the liposome of the present invention is not particularly limited, and an excessive amount of the compound can be reacted with the remaining maleimidated lipids. A preferred introduced amount of the polyalkylene glycol is about 0.28 to 0.90 mole%, more preferably about 0.28 to 0.56 mole%, based on the total lipids, about 15 to 50 mole%, more preferably about 15 to 30 mole%, based on the maleimidated lipids, and about 0.44 to 1.45 mole%, more preferably about 0.44 to 0.89 mole%, based on DPPC.

The liposome of the present invention may be modified with a protein, for example, an antibody. As a protein, for example, various physiologically active substances such as antibodies, FGF and EGF can be used. Preferably, antibodies can be used. As the antibody, an antibody per se or a fragment of an antibody, a derivative of an antibody or the like can be used, and either an antibody per se or an antibody fragment may be used as the antibody. The term "antibody" used in the specification encompasses derived or modified antibodies and the like as well as antibodies per se and fragments of antibodies, and the term should be interpreted in the broadest sense. As the antibody, an antibody having reactivity with tissues, cells, bacteria, viruses or the like, which are targets of therapeutic treatment, may also be used,. For example, polyclonal antibodies deriving from various animals, mouse monoclonal antibodies, human-mouse chimeric antibodies, human monoclonal antibodies and the like can be used. A human monoclonal antibody is more preferred, since the antibody is not a protein from a heterogenous animal. As the antibody, the human monoclonal antibody described in Japanese Patent Unexamined Publication (Kokai) No. 5-304987 (GAH antibody) can be preferably used. For example, a liposome modified with a GAH antibody can be prepared according to the method specifically described in Example 7 of the above publication. As described in Japanese Patent Unexamined Publication (Kokai) No. 5-304987, since the GAH antibody has reactivity with stomach cancer and colon cancer, the liposome of the present invention is useful as an anticancer agent for treatment of stomach cancer, colon cancer and the like. A liposome having an excellent therapeutic effect can be produced by appropriately choosing a combination of the type of an antibody and a medicament to be encapsulated.

After a thiol group is introduced to an antibody, a liposome can be modified with the antibody by reacting a maleimide group on the liposome with the thiolated antibody. The addition of a thiol group to the antibody is carried out by reacting a compound ordinarily used for thiolation of a protein such as N-succinimidyl-3-(2-pyridyldithio)propinate (SPDP) (Carlsson, J., et al., Biochem. J., 173, 723, 1978), iminothiolane or mercaptoalkylimidate (Traut, R.R., et al., Biochemistry, 12, 3266, 1973) with an amino group of the antibody. Further, an endogenous dithiol group of an antibody can be reduced and reacted as a thiol group. From a viewpoint of sustained antibody activity, the method of using an endogenous thiol group is preferred.

For example, when IgG is used, an F(ab')₂ fragment can be obtained by using an enzyme such as pepsin, and then a thiol group, generated in a Fab' fragment which is obtained by reducing the F(ab')₂ fragment by using dithiothreitol, can be utilized in the bonding reaction with a liposome (Martin, F.J. et al., Biochemistry, 20, 4229, 1981). Where IgM is used, a thiol group of an Fc region of IgMs obtained by reducing the J chain under a mild condition can be utilized for bonding with a liposome according to the method of Mirror et al. (J. Biol. Chem., 257, 286, 1965). When the GAH antibody described in Japanese Patent Unexamined Publication (Kokai) No. 5-304987 is used, an F(ab')₂ fragment is preferably used. Bonding of a protein such as an antibody added with a thiol group and a liposome containing a maleimide group is achieved by a reaction in a neutral buffer (pH 6.5 to 7.5) for 2 to 16 hours.

A liposome bonded with an antibody and a compound containing a polyalkylene glycol moiety is a most preferred embodiment of the present invention. To produce this liposome, a thiolated antibody is reacted with a liposome having a maleimide group in a neutral buffer. For example, thiolated antibodies in an amount of 0.1 mole% (specifically, 0.17 mole%) to about 2 mole% (specifically, 1.5 mole%, 1.8 mole%), preferably 0.4 to 0.7 mole%, can be reacted with one mole of the maleimide groups (maleimidated lipids) so that 0.5 to 5.3 mg, 0.5 to 4.5 mg, preferably 1.2 to 2 mg, of antibodies are bonded per 100 mg of total lipids constituting the liposome. Then, a compound containing a polyalkylene glycol moiety can be reacted with the remaining maleimide groups to prepare a liposome bonded with the antibody and the compound containing a polyalkylene glycol moiety. More specifically, 15 to 50 mole%, preferably 15 to 30 mole% of a compound containing a thiolated polyalkylene glycol moiety is added to one mole of maleimidated lipid groups (i.e., 0.28 to 0.90 mole%, preferably 0.28 to 0.56 mole% based on the total lipids, and when DPPC is used, 0.44 to 1.45 mole%, preferably 0.44 to 0.89 mole% based on DPPC) to produce a liposome bonded with the antibody and the compound containing a polyalkylene glycol moiety.

As a preferred embodiment of the liposome of the present invention, a liposome is provided, which encapsulates a medicament, preferably an antitumor agent such as doxorubicin, and is bonded with a compound containing a polyethylene glycol moiety and an antitumor antibody. The liposome containing a medicament can be formulated by a known method, for example, the dehydration method (International Patent Unexamined Publication in Japanese (Kohyo) No. 2-502348), the method wherein the liposome is added with a stabilizer and used as a liquid preparation (Japanese Patent Unexamined Publication (Kokai) No. 64-9331), the lyophilization method (Japanese Patent Unexamined Publication (Kokai) No. 64-9931) or the like. The liposome can be administered to a patient through intravascular administration, intravesical administration, intraperitoneal administration, local administration or the like for treatments of various diseases such as cancers. A dose can be appropriately chosen depending on a type of a medicament encapsulated as an active ingredient. For example, when a liposome encapsulating doxorubicin is administered, the liposome can be used in an amount of 50 mg/kg or less, preferably 10 mg/kg or less, more preferably 5 mg/kg or less, as a weight of an active ingredient.

### Examples

The present invention will be more specifically explained with reference to the examples. However, the scope of the present invention is not limited to these examples.

### Example 1

### (1) Preparation of a liposome and encapsulation of a medicament

A lipid mixture (1.6 g) of dipalmitoylphosphatidylcholine (DPPC)/cholesterol/ε-maleimidocaproyldipalmitoylphosphatidylethanolamine (MC-DPPE) (18:10:0.5 in molar ratio) was added with 16 mL of 0.3 M citrate buffer (pH 4.0) and hydrated. Then, freeze and thawing of the mixture was repeated three times using liquid nitrogen and a warm bath at 60°C to produce multilamellar liposomes. The particles were sized to 0.1 µm by the extrusion method. The resulting liposome solution was neutralized by adding 1 M NaOH dropwise. Then the solution was heated to 60°C and added with an aqueous doxorubicin solution (DXR, also referred to as adriamycin, ADM) at an amount of 0.5 mL of 20 mg/mL per 100 mg of lipids for encapsulation.

### (2) Thiolation of an antibody and bonding thereof to liposome (preparation of antibody-bonded liposome)

GAH antibody (a monoclonal antibody described in Japanese Patent Unexamined Publication (Kokai) Nos. 4-346918 and 5-304987 reactive to stomach cancer and colon cancer, 3 mg/mL, 14.4 mL) dissolved in 50 mM phosphate buffer (pH 7.5) containing 1 mM EDTA was added with 92.4 µL of 3 mg/mL iminothiolane and reacted at 37°C for 1 hour to introduce thiol groups (Biochemistry, 12, 3206, 1973). The reaction mixture was subjected to gel filtration and the buffer was exchanged with 0.1 M phosphate buffer (pH 6.0) containing 1 mM EDTA. Then, the resulting thiolated antibody (0.21 mg, 1.7 mg/mL) per 1 mg liposomal doxorubicin was reacted with the liposome at 25°C for 1 hour to form bonding of the antibody to the liposome.

### (3) Thiolation of polyethylene glycol and bonding to liposome (preparation of PEG-bonded liposome)

Cystine was reacted with 2,4-bis(polyethylene glycol)-6-chloro-s-triazine according to the method of Japanese Patent Unexamined Publication (Kokai) No. 4-346918 and then reduced to prepare polyethylene glycol (two-chain type PEG) having thiol groups. More specifically, thiolated PEG (30 mg/mL, a two-chain type PEG having a molecular weight of 2000 (PEG 2000) and a two-chain type PEG having a molecular weight of 5000 (PEG 5000)) were produced by using cystine, and each of them was added to the above reaction mixture in an amount of 0.18 mL per 1 mL of the reaction mixture for a bonding reaction at 10°C. After 5 to 240 minutes from the start of the reaction, the reaction mixture was sampled and applied to a Sepharose CL6B column to stop the reaction by removing unreacted thiolated PEG to obtain immunoliposomes having different PEG amounts. Non-PEG-bonded immunoliposomes were similarly prepared by subjecting immunoliposomes without the PEG binding treatment to gel filtration using Sepharose CL6B.

### (4) Preparation of liposome bonded with antibody and PEG

Liposomes bonded with antibodies and PEG were prepared by reacting the thiolated PEG prepared in the above (3) with the antibody-bonded liposomes prepared in the above (2).

### (5) Quantification of bonded PEG

The amount of PEG bonded to immunoliposomes was determined by HPLC. An immunoliposome solution prepared as a SDS solution at a final concentration of 2% was heated at 60°C for 30 minutes to completely solubilize the liposomes. The solution was eluted by using a GPC column (2000SWXL, Tosoh Corporation) with a buffer at pH 7.5 (25 mM NaH₂PO₄, 0.1% SDS, 70% methanol) to separate PEG. The PEG was detected at 215 nm and quantified using area values.

### (6) Quantification of DXR

50 µL of a sample was added to 950 µL of 50% propanol/0.5 M hydrochloric acid and absorption was measured at 500 nm to quantify DXR.

### (7) Quantification of bonded antibody

The antibodies were eluted and separated by using a GPC column (3000SWXL, Tosoh Corporation) with a buffer at pH 7.0 (25 mM NaH₂PO₄, 200 mM Na₂SO₄, 0.1% SDS) in the same manner as in the qualification of PEG. The antibodies were quantified using area values of the antibody peak detected at 280 nm.

### (8) Quantification of lipids

Lipids (total amount of DPPC and cholesterol) were quantified by HPLC. The liposomes were loaded on an L-column ODS (4.6 mm × 250 mm, Chemicals Inspection & Testing Institute) and lipids were eluted with tetrahydrofuran (THF)/acetonitrile/water (2:1:1 (v/v/v), 0.1% trifluoroacetic acid). The lipids were detected at 215 nm and quantified from area values of the peaks of DPPC and cholesterol. A sample for quantifying lipids was prepared by adding a 9-fold volume of the above eluent to one volume of the liposome sample.

### Example 2: Preparation of PEG-bonded liposome (pharmacokinetic test)

According to the method of Example 1, liposomes were prepared which were introduced with PEG in an amount of 0 to about 0.7 mole% based on one mole of the total lipids constituting the liposomes, 0 to about 1 mole% based on one mole of DPPC, or 0 to about 40 mole% based on one mole of maleimidated lipids (encapsulating DXR with no antibodies bonded, and bonded with PEG 2000 or PEG 5000).

**<Table 1>**

| Liposome | DXR concentration (mg/ml) | PEG amount* | PEG amount** | PEG amount*** |
|---|---|---|---|---|
| 1 | 1.14 | 0.083 (PEG 5000) | 0.13 | 4.61 |
| 2 | 1.17 | 0.22 (PEG 5000) | 0.34 | 12.2 |
| 3 | 1.27 | 0.40 (PEG 5000) | 0.63 | 22.2 |
| 4 | 1.32 | 0.48 (PEG 5000) | 0.75 | 26.7 |
| 5 | 1.16 | 0.096 (PEG 2000) | 0.15 | 5.33 |
| 6 | 1.08 | 0.30 (PEG 2000) | 0.47 | 16.7 |
| 7 | 1.47 | 0.60 (PEG 2000) | 0.94 | 33.3 |
| 8 | 1.55 | 0.70 (PEG 2000) | 1.09 | 38.9 |
| 9 | 3.49 | - | - | - |

| | | | | |
|---|---|---|---|---|
| * Mole % to total lipids | | | | |
| ** Mole % to DPPC | | | | |
| *** Mole % to maleimidated lipid DPPE | | | | |

Male BALB/cAjcl mice (6-week old) were used in experiments. A mouse group consisting of four mice was used for each of the liposomes, and one untreated mouse was used as a blank in the experiment. The liposomes were administered into the tail vein at the dose of 2 mg/kg as DXR. After 16 hours from the administration, a Nembutal injection solution (Dainippon Pharmaceutical Co., Ltd.) was intraperitoneally administered to the mice and thoracotomy was performed under anesthetization to collect blood. Blood plasma was separated and used for analysis of DXR.

100 µL of the plasma was added with 1 mL of 0.3 M hydrochloric acid/50% ethanol (hydrochloric ethanol) and heated at 60°C for 10 minutes to extract DXR. The extract was cooled to 4°C and centrifuged at 15,000 rpm for 10 minutes to collect a supernatant. The sample was diluted 4 times with hydrochloric ethanol and its fluorescence was measured at a fluorescence wavelength of 590 nm with an excitation wavelength of 490 nm. A calibration curve was prepared by using DXR diluted with hydrochloric ethanol to known concentrations and used in quantification of DXR in plasma. The recovery of DXR from the mouse plasma was 95 to 98% in a range of 3.75 to 30 µg/mL of DXR (Reference: Cancer Res., 47, 4471, 1989).

The average values (± standard deviation) of plasma DXR concentrations in the respective liposome groups are shown in Fig. 1. It was found that the plasma DXR concentration as an index of retention of the liposomes in blood increased depending on the amount of PEG bonded to the liposomes for both of PEG 5000 and PEG 2000.

### Example 3: Preparation of liposome bonded with PEG and antibodies (pharmacokinetic test)

Liposomes introduced with PEG in an amount of 0 to 0.6 mole% based on the total lipids in the liposome, 0 to about 1 mole% based on DPPC and 0 to about 30 mole% based on maleimidated DPPE (encapsulating DXR, bonded with antibodies and PEG 5000) were prepared according to the method of Example 1.

**<Table 2>**

| Liposome | DXR concentration (mg/100 mg lipids) | Antibody (mg/100 mg lipids) | PEG amount* | PEG amount** | PEG amount*** |
|---|---|---|---|---|---|
| 1 | 10.2 | 1.5 | 0 | 0 | 0 |
| 2 | 10.4 | 1.6 | 0.06 | 0.10 | 3 |
| 3 | 10.3 | 1.6 | 0.10 | 0.16 | 5 |
| 4 | 10.3 | 1.6 | 0.15 | 0.24 | 8 |
| 5 | 11.1 | 1.6 | 0.28 | 0.44 | 15 |
| 6 | 10.5 | 1.6 | 0.30 | 0.48 | 16 |
| 7 | 10.2 | 1.6 | 0.36 | 0.56 | 19 |
| 8 | 11.0 | 1.8 | 0.48 | 0.76 | 26 |
| 9 | 10.8 | 1.3 | 0.52 | 0.82 | 28 |
| 10 | 10.8 | 1.7 | 0.61 | 0.97 | 33 |

| | | | | | |
|---|---|---|---|---|---|
| * Mole% based on total lipids | | | | | |
| ** Mole% based on one mole of DPPC | | | | | |
| *** Mole% based on one mole of maleimidated lipid DPPE | | | | | |

DXR in blood plasma was quantified in the same manner as in Example 2. The average values (± standard deviation) of plasma DXR concentrations in the respective liposome groups are shown in Fig. 2. The plasma DXR concentration increased depending on the amount of PEG bonded to the liposomes, but it was found that the concentration reached a plateau at an introduced amount of PEG of about 0.3 mole% based on the total lipids, about 0.5 mole% based on DPPC, and about 17 mole% based on maleimidated DPPE. When DXR leaked from the liposomes, the leaked DXR was verified to rapidly disappear from blood, and accordingly, it is considered that the plasma DXR concentrations shown in the results in Examples 2 and 3 were attributable to DXR encapsulated in the liposomes.

### Example 4: Preparation of liposome bonded with PEG and an antibody (efficacy test, pharmacokinetic test)

The following Liposomes 2 to 7, in which 0.5, 1.2, 2.0, 4.5, 5.3 and 11.4 mg of antibodies were bonded to 100 mg of the total lipids of the liposome (encapsulating DXR , bonded with PEG 5000), were prepared according to the method of Example 1. In a similar manner, Liposomes 1 bonded with no antibody were prepared. In the PEG-bonded liposomes, retention of each liposome in blood was equivalent within the range of the amount of PEG bonded (> 4.4 mg/100 mg lipids). Therefore, the experimental results shown below depended on the bonded amount of antibodies.

**<Table 3>**

| Liposome | Amount of bonded antibodies (mg/100 mg lipids) | Amount of included DXR (mg/100 mg lipids) | Amount of bonded PEG (mg/100 mg lipids) |
|---|---|---|---|
| 1 | 0 | 9.5 | 8.2 |
| 2 | 0.5 | 9.1 | 8.2 |
| 3 | 1.2 | 9.5 | 8.1 |
| 4 | 2.0 | 8.9 | 5.3 |
| 5 | 4.5 | 9.6 | 6.2 |
| 6 | 5.3 | 9.7 | 6.4 |
| 7 | 11.4 | 10.0 | 3.2 |

Stomach cancer cell strain MKN45 was subcutaneously transplanted at two sites (1 × 10⁶ cells per site) on the dorsal side of nude mice. For the "efficacy test", administrations of liposomes with different amounts of bonded antibodies were started when the tumor reached to a size large enough to measure its long and short diameters. The dose of the liposomes was 5.0 mg/kg (as the amount of DXR) per administration. A DXR-administered group (5.0 mg/kg) was provided as a positive control, and physiological saline was administered to the control group. All of the groups were intravenously administered on the day of the start of the administration (day 0), day 3 and day 6.

The diameters of the tumors (longer and shorter diameters) were measured with time and an estimated tumor weight ((shorter diameter)² × longer diameter/2) was calculated. After the three times of administration, the measurement was continued until day 19. The tumor proliferation rate was calculated based on the estimated weight of each tumor on the day of the start of the administration, which was taken as 1.0 to evaluate inhibitory effect on tumor proliferation of each treated group relative to the control group and the DXR-administered group. On the last day of the measurement (day 19), a %T/C value (tumor proliferation rate of treated group/tumor proliferation rate of control group × 100) was calculated for each treated group. The amount of bonded antibodies which achieved the maximum inhibitory effect against tumor proliferation (an optimum dose) was obtained based on the amount of bonded antibodies in each sample.

As a result, significant inhibitory effects against tumor proliferation were found in all of the treated groups compared with the control group. As shown in Fig. 3, when comparison was made to the DXR-administered group, significant inhibitory effects against tumor proliferation were observed in the samples with the amounts of bonded antibodies within the range of 0.5 to 5.3 mg/100 mg of total lipids. The inhibitory activity against tumor proliferation was observed with a peak in the vicinity of 2 mg/100 mg of total lipids as the amount of bonded antibodies.

In the "pharmacokinetic test", the above Liposomes 4 to 7 with different amounts of bonded antibodies (2.0, 4.5, 5.3 and 11.4 mg/100 mg of total lipids) were intravenously administered to mice (each group consisted of 2 or 3 mice, 1.0 mg/kg as the amount of DXR amount). Four hours after the administration, blood plasma was collected from each animal. The amount of DXR in plasma was measured by the fluorescence measurement method in the same manner as in Example 2. The amounts of DXR in plasma in the respective samples after the administration were compared to found correlation between the amount of bonded antibodies and the retention in blood of the liposomes encapsulating DXR and bonded with antibodies. In the pharmacokinetic test, correlation between the DXR, amount in plasma after administration of each sample and the amount of bonded antibodies of each sample was obtained for samples having the amount of the bonded antibodies of 2 mg/100 mg of lipids or more (Fig. 4). As a result, it was found that, when the amount of the bonded antibodies exceeded 2 mg/100 mg of the lipids, the retention in blood decreased depending on the increasing amount of the bonded antibodies.

### Industrial Applicability

The liposome of the present invention is characterized by excellent retention in blood and a reduced amount of bonded polyalkylene glycol compared to conventional liposomes. Therefore, the liposome of the present invention can avoid side effects caused by polyalkylene glycol and has advantages from a viewpoint of industrial productivity. The liposome of the present invention is further characterized that the liposome can achieve more excellent therapeutic effect compared to conventional antibody-bonded liposomes.

## Claims

1. A liposome wherein a compound containing two polyalkylene glycol groups is bonded through a thioether group to a liposome comprising lipids whose partial component has maleimidated terminal, and wherein an amount of the bonded compound is 15 to 30 mole% based on one mole of the maleimidated lipid contained in the liposome.

2. The liposome according to claim 1, which is obtained by reacting a maleimide group of the maleimidated lipid with the compound containing two polyalkylene glycol groups introduced with a thiol group.

3. The liposome according to any one of claims 1 to 2, wherein the compound is bonded to a surface of the liposome.

4. The liposome according to any one of claims 1 to 3, wherein the polyalkylene glycol is polyethylene glycol.

5. The liposome according to claim 4, wherein the compound has two polyethylene glycol groups.

6. The liposome according to claim 5, wherein the polyethylene glycol has a molecular weight of 2,000 to 7,000 daltons.

7. The liposome according to claim 5, wherein the polyethylene glycol has a molecular weight of about 5,000 daltons.

8. The liposome according to any one of claims 1 to 7, wherein an antibody is further bonded on a surface of the liposome.

9. A medicament suitable for treatment of cancer according to any one of claims 1 to 8.

10. The medicament according to claim 9, wherein the cancer is stomach cancer or colon cancer.

11. Use of the liposome according to any one of claims 1 to 10 for the preparation of a medicament for the treatment of cancer.
